# EUROPEAN PATENT APPLICATION

(11) **EP 3 626 305 A1**
(43) Date of publication of application: **25.03.2020**
(21) Application number: 18199361.9
(22) Date of filing: 09.10.2018
(51) Int. Cl.: A61N 1/372, A61N 1/375, H01Q 1/36, H01Q 1/27, H01Q 9/42, H01Q 23/00

(54) **ELECTRICAL ARRANGEMENT FOR INDUCTIVE CHARGING AND WIRELESS COMMUNICATION**

(30) Priority: 21.09.2018 US 201862734287 P
(71) Applicant: BIOTRONIK SE & Co. KG, 12359 Berlin (DE)
(72) Inventor: Wang, Yu, Lake Oswego, OR Oregon 97035 (US); Brown, James E., Tigard, OR Oregon 97224 (US); von Arx, Jeffrey A., Lake Oswego, OR Oregon 97035 (US); Stadnik, Paul, Lake Oswego, OR Oregon 97035 (US); McIntosh, David, Wilsonville, OR Oregon 97070 (US); Muessig, Dirk, West Linn, OR Oregon 97068 (US)
(74) Representative: Keck, Hans-Georg

(57) **Abstract**

This disclosure is directed to an electrical arrangement for inductive charging and wireless communication, a header structure comprising the electrical arrangement and an implantable medical device (IMD) comprising the header structure. The electrical arrangement comprises a charging coil configured for inductive charging, wherein the charging coil comprises at least a first connection line; an antenna structure configured for wireless communication, wherein the antenna structure is formed of at least a part of the first connection line, and wherein the charging coil and the antenna structure are electrically connected in series.

## Description

This invention generally relates to inductively rechargeable implantable devices having a device header portion and means serving as telemetry antenna and electrical circuitry provides therefor.

In the field of implantable devices, it is desirable to minimize the space of the device in order to enhance patient comfort, simplify the implantation procedure and to reduce the risk of post-operational infections.

Implantable electrical devices having the capability of sensing physiological parameters and/or the capability of electrical stimulation of nervous tissue typically comprise of a device housing made of metallic material, and a device header portion whose body is primarily made primarily of a non-metallic material. The main electronic components are situated within the device housing, wherein electrical feedthroughs are implemented at the interface between device housing and device header portion in order to provide electrical connection between electrical components inside the device housing and electrical components outside the device housing, as for instance lead electrodes or the like. Feedthroughs are costly and critical components regarding reliability of signal transmission, tightness to fluids.

Therefore, it is desirable to keep the number of feedthroughs as small as possible.

Some implantable electrical devices are inductively rechargeable via an external inductive charging coil. Such devices have an enhanced longevity, eliminating the necessity of explantation due to a depleted battery. Situating the inductive charging coil in the device housing has disadvantages of a slow recharging process due to losses in the conductor and heating due to eddy currents in the device housing material.

Moreover, state of the art implantable electrical devices typically have telecommunication means, as for instance means configured as telemetry antenna for transmitting signals to / receiving signals from another implantable device, an external device, an external data center or the like. It is desirable to obtain high transmission and receiving performance of the antenna.

US 9,403,024 B2 describes an implantable device having a coil which is capable of performing both communication and charging functions. The coil is attached to a support structure that is located within the device case.

Due to placement of the charging and telemetry coil within the device case, drawbacks of the device in US 9,403,024 B2 lie in reduced transmission and receiving performance of the antenna, a slow recharging process and heating of the device case during recharging.

US 9,750,930 B2 discloses an implantable device with a coil inside the header, wherein the coil is used for both inductive charging and as RF antenna. The device comprises inductive charging circuitry configured to provide electrical power to the medical device via the inductive charging signals and telemetry circuitry configured to conduct telecommunications with one or more external devices via the telemetry signals. Moreover, the device comprises a first component that is electrically coupled between the coil and the inductive charging circuitry, wherein the first component is configured to allow the inductive charging signals to pass through, and a second component that is electrically coupled between the coil and the telemetry circuitry, wherein the second component is configured to substantially block the inductive charging signals while allowing the telemetry signals to pass through.

Using one coil for both recharging and telemetry results in degraded RF performance for telemetry transmissions for the devices in US 9,750,930 B2 and US 9,403,024 B2, particularly because the electrical impedance of the recharge coil is unsuitable for acting as antenna transceiver and vice versa. Using one coil for both purposes therefore results in losses in recharging performance and/or telemetry performance.

US 9,634,381 B2 describes a device that includes radio frequency (RF) communication components installed within a case of the device and an antenna with an inverted E shape mounted within a header of the device. The antenna has three branches extending from a main arm: a capacitive branch connecting one end of the main arm to the case; an RF signal feed branch connecting a middle portion of the main arm to the internal RF components of the device via a feedthrough; and an inductive branch connecting the opposing (far) end of the main arm to the case to provide a shunt to ground.

EP 2 588 191 B1 describes a three-dimensional antenna that may be used for an implantable medical device (IMD). The antenna includes a first antenna portion that includes a plurality of segments arranged substantially parallel to one another in a first plane. The antenna further includes a second antenna portion that includes a plurality of segments arranged substantially parallel to one another in a second plane that is substantially parallel to the first plane. The antenna further includes a third antenna portion that includes a plurality of segments arranged substantially parallel to one another in a third plane. The plurality of segments of the third portion are coupled between segments of the first and second portions. The third plane is arranged substantially perpendicular to the first plane and the second plane.

It is an objective of the present invention to solve the problems stated above.

In particular, it is an objective of the present invention to provide an inductively rechargeable IMD with telemetry function, wherein charging and telemetry are performed in an efficient manner, and wherein the volume of the IMD and the number of critical components as e.g. feedthroughs is kept low.

According to the invention, an electrical arrangement for inductive charging and wireless communication is proposed, comprising:
- a charging coil configured for inductive charging, wherein the charging coil comprises at least a first connection line,
- an antenna structure configured for wireless communication, wherein the antenna structure is formed of at least a part of the first connection line,
- wherein the charging coil and the antenna structure are electrically connected in series.

An advantage of a series connection of charging coil and antenna structure is that less connection lines are required than e.g. arranging charging coil and antenna in parallel. As a result, less feedthroughs are required for coupling the electrical arrangement to other electronics.

A further advantage of a series connection of charging coil and antenna structure is that inductive charging function of the coil and signal transmission and receiving function of the antenna structure can be performed in a more effective manner. Compared to using one coil for both purposes, the electrical characteristics of charging coil and the antenna structure are optimized for their dedicated purpose. Moreover, electrical impedance of the antenna structure may be varied via impedance matching, i.e. in consideration of the charging coil or not, allowing improved signal transmission/reception in the desired frequency band. According to an embodiment, the geometry flexibility of series connection of antenna and coil offers multiple modes of far field radiation at multiple frequencies.

Preferably, according to an aspect of the invention, the charging coil comprises a second connection line, wherein the antenna structure is formed of at least a part of the first connection line and at least a part of the second connection line. At least one end of the first connection line is configured to be connected to a first feedthrough and/or wherein at least one end of the second connection line is configured to be connected to a second feedthrough.

By implementing the antenna structure as connection line of the charging coil or part of the same, the series connection of charging coil and antenna structure can be practically put into practice, allowing a space-saving solution for the electrical arrangement according to an aspect of the invention. The antenna structure can be implemented as part of one or more than one connection line of the charging coil.

For instance, the antenna structure and a section of the charging coil are configured as a loop. In an embodiment of the invention, the antenna structure and a section of the charging coil are adapted for Industrial, Scientific and Medical (ISM) band far field radiation.

According to a preferred embodiment of the present invention, the antenna structure comprises at least a part which is straight and/or a part which has a meandering shape, a spiral shape. Furthermore, the antenna can be a patch antenna, a conformal patch antenna, a fractal antenna or a helical antenna. For the present invention and embodiments thereof, it is understood that the described shapes of antenna structure are related to the shape of the path an electrical current takes through the antenna structure.

According to an embodiment of the present invention, the charging coil, the antenna structure and a feedline of the antenna structure are in combination may be configured similar to an inverted F antenna (IFA). It is understood for the present invention and embodiments thereof, that "similar to an inverted F antenna" is interpreted that the main parts of an IFA can be found in a radiation structure. The main parts are regarded as monopole antenna, feedline and a shorting pin at one end of the antenna.

According to an embodiment of the invention, the charging coil, a connection line of the charging coil whereby the antenna structure is implemented at least as part of the connection line form a radiation structure similar to an IFA. At high RF frequencies the many turns of wire that the coil consists of (copper in the preferred embodiment) are all capacitively shorted together. This results in the coil electrically acting as a solid metal enlongated torus in the device header. At high frequencies (as for instance at frequencies of the MICS or ISM band), this torus itself can be treated as the RF ground. Particularly, the coil is treated as RF ground when the radiation elements of the antenna are driven single ended. In case the radiation elements are driven symetrically from dual feedlines, the coil is treated as virtual floating arm. According to an embodiment, the antenna structure corresponds to the monopole antenna, where by the antenna is configured as ½ wavelength monopole ensuring a voltage of 0 at the ground connection. connection lineFor instance, said radiation structure can be optimized for MICS band operation.

According to an aspect of the invention, the antenna structure is configured to receive and/or transmit radiofrequency signals associated with the frequency band of the Medical Implant Communication Service (MICS) spectrum. Specifically, the antenna structure is configured to receive and/or transmit radiofrequency signals at approximately 403 MHz.

Preferably, the antenna structure is configured to receive and/or transmit radiofrequency signals associated with the frequency band of the Industrial, Scientific and Medical (ISM) spectrum. Specifically, the antenna structure is configured to receive and/or transmit radiofrequency signals at approximately 2,4 GHz. According to embodiments, the 902 to 928 MHz ISM band or the 868 MHz SRD (Short Range Devices) band is used.

According to an embodiment of the present invention, the antenna structure is configured to receive and/or transmit radiofrequency signals at least at a first frequency band and a second frequency band, wherein the first frequency band differs from the second frequency band. According to an embodiment, the first frequency band may be the MICS band and the second frequency band may be the ISM band.

According to an embodiment of the present invention, the at least first connection line is connected to an RF matching circuit. According to an embodiment, the RF matching circuit comprises at least one of the following components:
- resistor,
- capacitor,
- inductor
- λ/4 transmission line.

Preferably, according to an embodiment of the invention, the first connection line is connected to a receiver exchange matching circuit or a transmission exchange matching circuit and wherein the second connection line is connected to a receiver exchange matching circuit.

According to an aspect of the present invention, at least one of the first connection line or the second connection line is connected to a λ/4 impedance transmission line. A λ/4 impedance transmission line provides advantages for adapting transmission line characteristics of the circuit.

According to an aspect of the invention, at least one of the first connection line or the second connection line is connected to at least one of or a combination of:
- a radiofrequency transceiver,
- a receiver exchange matching circuit,
- a transmission exchange matching circuit, and/or
- an inductive transceiver and charging circuit.

Moreover, according to an embodiment of the present invention, the electrical arrangement further comprises a plurality of capacitors, wherein the capacitors are connected in series and/or in parallel to at least one of or a combination of
- the radiofrequency transceiver,
- the receiver exchange matching circuit,
- the transmission exchange matching circuit, and/or
- the inductive transceiver and charging circuit,
wherein the capacitors are configured to control at least one of or a combination of
- radiofrequency impedance of an inductive interface of the charging coil,
- resonance and/or impedance of the charging coil, and/or
- radiofrequency impedance and resonance of the antenna structure.

For example, the first connection line is connected to an RF matching circuit, wherein the RF matching circuit is connected to an RF transceiver unit. A capacitor for RF matching may be implemented between first connection line and RF matching circuit. According to an embodiment, the second connection line is connected to the RF matching circuit. For instance, a capacitor for RF matching is implemented between second connection line and RF matching circuit.

According to an embodiment of the present invention, the first connection line is connected to a receiver exchange (Rx) matching circuit. For example, a capacitor for RF matching is implemented between first connection line and Rx matching circuit. According to an embodiment, the second connection line is connected to a transceiver exchange (Tx) matching circuit. For example, a capacitor for RF matching is implemented between second connection line and Tx matching circuit. For instance, Rx matching circuit and Tx matching circuit are connected to an RF transceiver.

The capacitors between the first and/or second connection line and the RF matching circuit, the Rx matching circuit or the Tx matching circuit can be varied in size and/or location in order to control the RF impedance and resonance of the antenna structure.

Moreover, according to an embodiment of the present invention, the first connection line is connected to an inductive transceiver and charging circuit which is configured to process low frequency currents for charging e.g. a battery. For instance, capacitors are implemented between first and/or second connection line and the inductive transceiver and charging circuit in series. Size and location of the capacitors connected in series may be varied for controlling the resonance and impedance of low frequency coil. Moreover, according to an embodiment, capacitors may be connected parallel to the inductive transceiver and charging circuit. By varying size and location of the capacitors in parallel connection, RF impedance of an inductive interface of the charging coil can be controlled.

Moreover, the present invention proposes a header structure for an implantable medical device, wherein the header structure comprises said electrical arrangement.

Preferably, according to an aspect of the present invention, the header structure comprises a header body, wherein the charging coil and the antenna structure of the electrical arrangement are arranged within the header body.

Arranging the serially connected charging coil and antenna structure within the header body allows highly efficient charging and telemetry functionalities.

For instance, if the header belongs to an implantable device having stimulation capabilities using stimulation leads, other components located within the header body may be connector modules for mechanical and electrical connection of stimulation leads. Moreover, electrical wires running from the feedthroughs to the electrical contacts of the connector modules are located within the header body.

For instance, the header structure is at least partly molded from a polymer material, as for instance epoxy resin, liquid crystal polymer (LCP), silicone, polysulfon, or a polyurethane (e.g. Tecothane^{tm}).

According to an aspect of the present invention, an implantable medical device is proposed which comprises said header structure. The implantable device further comprises a device housing, wherein the first feedthrough and/or the second feedthrough are arranged between header structure and the device housing.

Furthermore, according to an embodiment of the present invention, the first connection line and/or the second connection line are electrically connected with electrical components within the device housing of the implantable device via the first feedthrough and/or second feedthrough.

Preferably, according to an embodiment, at least one of the following structures or a combination thereof is implemented within the device case:
- a radiofrequency transceiver,
- a receiver exchange matching circuit,
- a transmission exchange matching circuit,
- an inductive transceiver and charging circuit, and/or
- a λ/4 impedance transmission line to which at least one of the first connection line or the second connection line is connected.

According to an embodiment, the λ/4 transmission line is part of a distributive filter network that creates high impedance of otherwise low impedance shunt capacitor that forms the resonant frequency with the charging coil.

In one embodiment, the IMD is a device with electrical measuring and therapy functions, as for example a cardiac pacemaker, an implantable defibrillator or a cardiac resynchronization device. In further examples, the IMD is a neuro stimulator as for example a spinal cord stimulator (SCS), vagus nerve stimulator (VNS), deep brain stimulator (DBS), or the like. The IMD may be a monitoring device without therapy function, as for example an implantable cardiac loop recorder. The IMD may have measurement and/or stimulation electrodes. The electrodes are for instance implemented on the device body, as it is known for instance for implantable cardiac loop recorders or miniature cardiac pacemakers without leads (also known as leadless pacemakers). Alternatively, the electrodes may be implemented on leads, wherein the lead comprises a lead body and a lead connector, which is configured to be plugged into a cavity of the device header for establishing mechanical and electrical connection to the IMD.

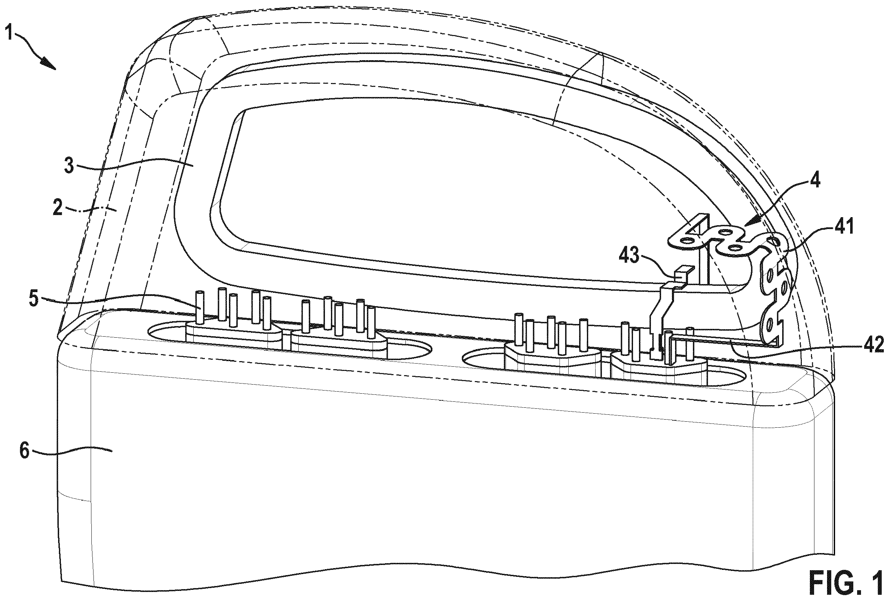

According to embodiments of the invention, an implantable device with a charging coil within the header is proposed. Radiation elements which are added to the feeding line of the charging coil serve as antenna structure for RF telemetry. The charging coil and the antenna are connected in series, whereby the number of feedthroughs can be reduced. Due to impedance transformation of the λ/4 transmission line and shunt capacitor(s) at the telemetry frequency and the high pass nature of the impedance match of telemetry antenna,, the inductive signals are not perturbing the telemetry signals and vice versa.
- Fig. 1: shows an exemplary embodiment of the electrical arrangement according to the invention,
- Fig. 2: shows a schematic diagram of an exemplary embodiment of the present invention where charging coil and antenna structure are in a loop configuration,
- Fig. 3: shows a schematic diagram of an exemplary embodiment of the present invention where charging coil and antenna structure are in a monopole configuration,
- Fig. 4: shows a schematic diagram of an exemplary embodiment of the present invention with dual feeds with independent Rx and Tx matching.
Referring to Fig. 1, a preferred embodiment of the present invention is schematically depicted. In this embodiment, IMD 1 is shown having a header structure 2, charging coil 3, antenna structure 4 serving as first connection line, second connection line 43, Four feedthrough assemblies 5, each having five feedthrough pins and device case 6. In this embodiment, antenna structure 4 represents a first connection line for charging coil 3, mechanically and electrically connecting charging coil 3 with a first feedthrough pin, wherein second connection line 43 connects an end of the charging coil mechanically and electrically with a second feedthrough pin. The electrical arrangement according to the invention is construed of charging coil 3 and serially connected antenna structure 4. Embodiments of the present invention are construed of variations, combinations and additions to the aforesaid structure. Antenna structure 4 comprises a part 41 having a meandering shape and a straight part 42. Charging coil 3 and antenna structure 4 are connected in series and therefore are connected to the device circuitry in the device case via a first feedthrough pin connected to straight part of the antenna 42 and a second feedthrough pin connected to second connection line 43. Second connection line 43 can be constructed as clamp or clip out of electrically conducting material, wherein the second connection line 43 is constructed to provide mechanical support of the charging coil within header 2 and electrical connection to a second feedthrough pin.

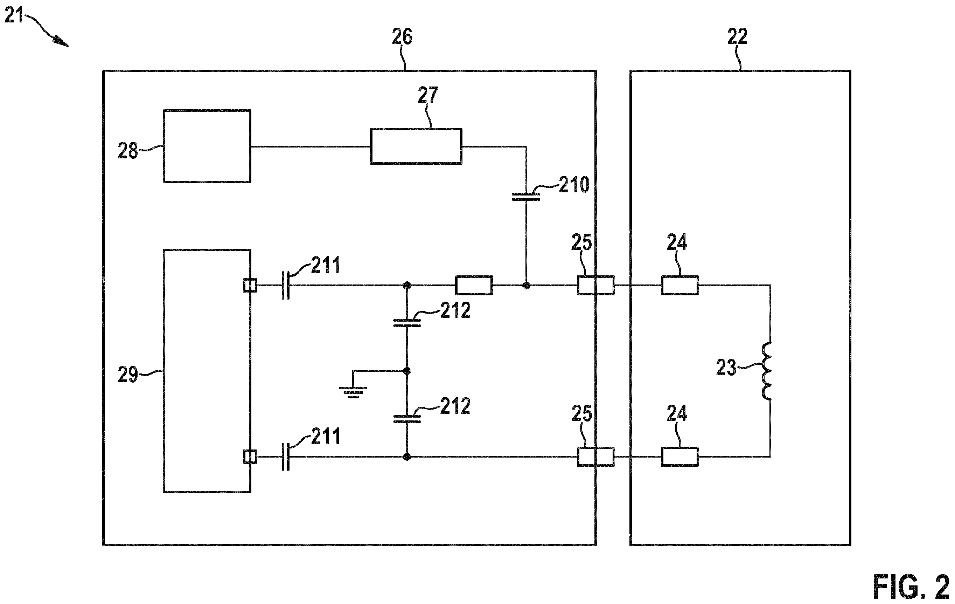

Fig. 2 shows an example of a schematic diagram of an exemplary embodiment of the present invention where charging coil and antenna structure are in a loop configuration. IMD 21 comprises housing structure 22, charging coil 23, antenna structure 24 which is in this embodiment construed as part of the first connection line and the second connection line of charging coil 23. Antenna structure 24 can for instance comprise at least one part with a meandering shape or a straight part. IMD 21 comprises furthermore feedthroughs 25 and device case 26. The electrical arrangement according to the invention is construed of charging coil 23 and serially connected antenna structure 24. Embodiments of the present invention are construed of variations, combinations and additions to the aforesaid structure. According to this embodiment, the antenna structure 24 can support operation at multiple RF frequencies such as one at MICS band and one at ISM band by altering the electrical length with and without the inductive coil. For instance, the antenna structure 24 and a small section of the charging coil 23 is configured as a small loop for ISM band far field radiation. Charging coil 23, and a connection line of the charging coil construed as antenna structure 24 form a radiation structure similar to an IFA. IMD 21 may comprise RF matching circuit 27 which is connected to an RF transceiver 28. One connection line of charging coil 23 / one arm of the antenna structure 24 may be electrically connected to RF matching circuit 27 e.g. via RF matching capacitor 210. IMD 21 further comprises Inductive transceiver and charging circuit 29 and for instance capacitors 211 for controlling the resonance and impedance of low frequency coil and/or capacitors 212 for controlling RF impedance of an inductive interface of the charging coil.

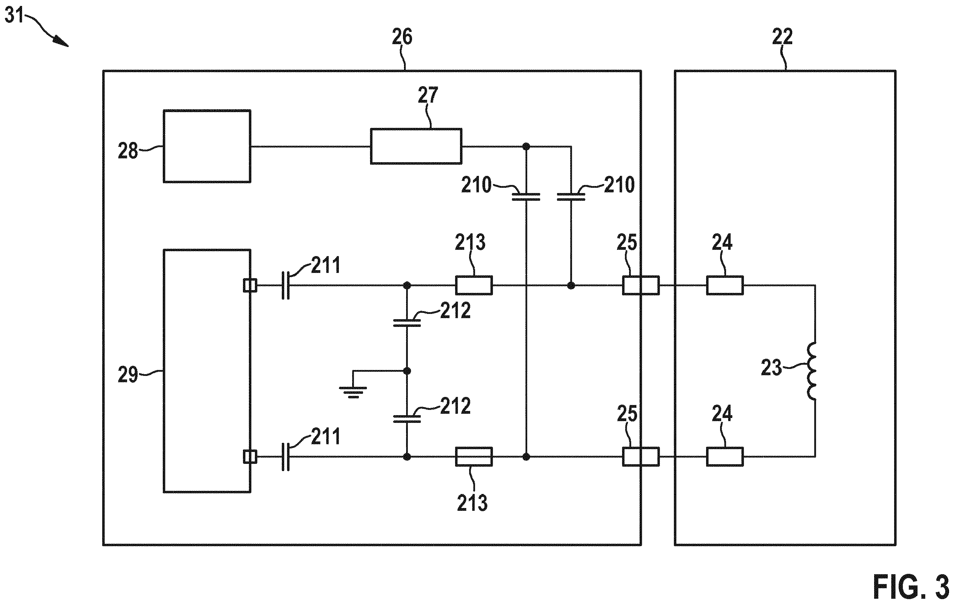

Fig. 3 shows a schematic diagram of an exemplary embodiment of the present invention where charging coil and antenna structure are in a monopole configuration. IMD 31 comprises housing structure 22, charging coil 23, antenna structure 24 which is in this embodiment construed as part of the first connection line and the second connection line of charging coil 23. Antenna structure 24 can for instance comprise at least one part with a meandering shape or a straight part. IMD 31 comprises furthermore feedthroughs 25 and device case 26. The electrical arrangement according to the invention is construed of charging coil 23 and serially connected antenna structure 24. Embodiments of the present invention are construed of variations, combinations and additions to the aforesaid structure. IMD 31 may comprise RF matching circuit 27 which is connected to an RF transceiver 28. Both connection lines of charging coil 23 / both arms of antenna structure 24 may be electrically connected to RF matching circuit 27 e.g. via RF matching capacitors 210. IMD 21 further comprises Inductive transceiver and charging circuit 29 and for instance capacitors 211 for controlling the resonance and impedance of low frequency coil and/or capacitors 212 for controlling RF impedance of an inductive interface of the charging coil. Moreover, IMD 31 comprises at least one λ/4 impedance transmission line element 213 or discrete blocking inductors (not shown). According to this embodiment, the two parts of the antenna structure 24 are driven by a common terminal from RF transceiver 28, wherein charging coil 23 is designed to be part of the antenna structure 24. The charging coil 24 operates typically at relatively lower frequencies as primary radiation element. The connection lines of the charging coil 24 and charging coil 24 function like a monopole antenna.

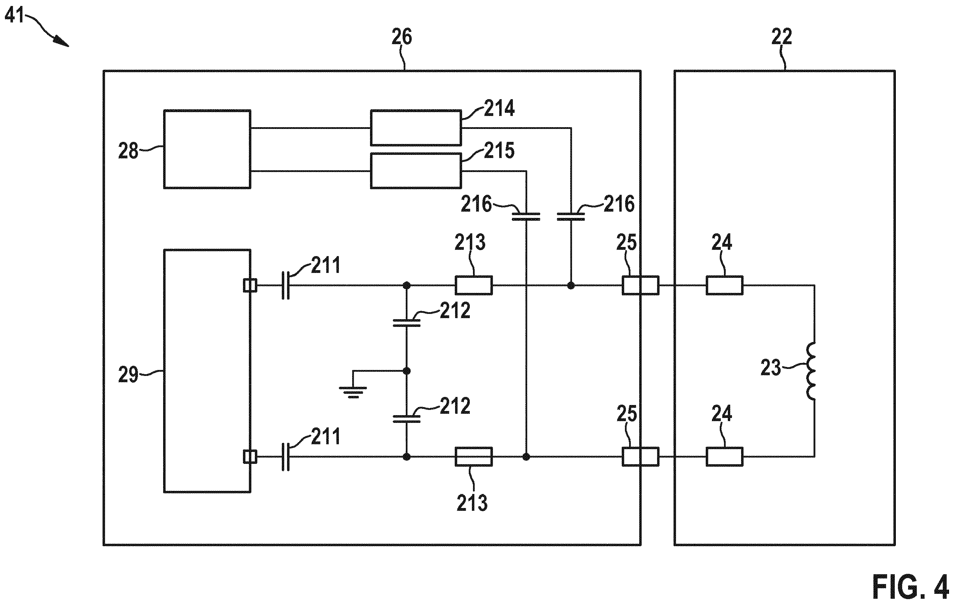

Fig. 4 shows a schematic diagram of an exemplary embodiment of the present invention with dual feeds with independent Rx and Tx matching. IMD 41 comprises housing structure 22, charging coil 23, antenna structure 24 which is in this embodiment construed as part of the first connection line and the second connection line of charging coil 23. Antenna structure 24 can for instance comprise at least one part with a meandering shape or a straight part. IMD 41 comprises furthermore feedthroughs 25 and device case 26. The electrical arrangement according to the invention is construed of charging coil 23 and serially connected antenna structure 24. Embodiments of the present invention are construed of variations, combinations and additions to the aforesaid structure. IMD 41 may comprise Rx matching circuit 214 and Tx matching circuit 215 which are connected to an RF transceiver 28. One connection line of charging coil 23 / one arm of antenna structure 24 may be electrically connected to Rx matching circuit 214, wherein the second connection line of charging coil 23 / the second arm of antenna structure 24 may be electrically connected to Tx matching circuit 215 e.g. via matching capacitors 216. IMD 41 further comprises Inductive transceiver and charging circuit 29 and for instance capacitors 211 for controlling the resonance and impedance of low frequency coil and/or capacitors 212 for controlling RF impedance of an inductive interface of the charging coil. Moreover, IMD 31 comprises at least one λ/4 impedance transmission line element 213. According to this embodiment, one of the arms of the antenna structure 24 is driven by the transmitter, and the second arm is connected to the receiver circuitry. For transceivers with separate Rx and Tx ports, this is the preferred configuration as the Rx path and Tx path can be independently optimized to achieve best RF performance and antenna efficiency. Also using the two connection lines / two arms of antenna structure 24 for separating Rx and Tx paths eliminates the need for a T/R (Transceiver/Receiver) switch in the transceiver design, reducing circuit complexity.

### List of Abbreviations

- IMD: Implantable Medical Device
- IFA: Inverted F Antenna
- MICS: Medical Implant Communication Service
- ISM: Industrial, Scientific and Medical
- RF: Radiofrequency
- Rx, Tx: Receiver Exchange, Transceiver Exchange

### Term definitions

- Meandering shape: Shape comprising at least one part forming an "S". Synonyms: Serpentine shape, S shape.
- Spiral shape: Shape comprising at least one winding or loop. Synonyms: Coiled, wound, helical shape.
- Connection line: Conducting element which connects electrical component directly or indirectly with energy storage, e.g. battery. In connection with an antenna, a connection line can be understood as feed line that connects the antenna with the radio transmitter or receiver.
- Connect, connected to/ coupled, coupled to: Electrically: Having an electrical conductive connection to (directly or with electrical components in between). Mechanically: Being in physical contact with

### List of Reference Signs

- 1, 21, 31, 41: IMD
- 2, 22: Header structure
- 3, 23: Charging coil
- 4, 24: Antenna structure
- 41: Part of antenna structure having meandering shape
- 42: Straight part of antenna structure
- 43: Second connection line
- 5, 25: Feedthrough assembly / Feedthroughs
- 6, 26: Device case
- 27: RF matching circuit
- 28: RF transceiver
- 29: Inductive transceiver and charging circuit
- 210: RF matching capacitor
- 211: Capacitors for controlling the resonance and impedance of low frequency coil
- 212: Capacitors for controlling RF impedance of an inductive interface of the charging coil
- 213: λ/4 impedance transmission line
- 214: Rx matching circuit
- 215: Tx matching circuit
- 216: Matching capacitors

## Claims

1. Electrical arrangement for inductive charging and wireless communication, comprising:
- a charging coil (3, 23) configured for inductive charging, wherein the charging coil comprises at least a first connection line,
- an antenna structure (4, 24) configured for wireless communication, wherein the antenna structure (4, 24) is formed of at least a part of the first connection line,
- wherein the charging coil (3, 23) and the antenna structure (4, 24) are electrically connected in series.

2. Electrical arrangement according to claim 1, wherein the charging coil (3, 23) comprises a second connection line, wherein the antenna structure (4, 24) is formed of at least a part of the first connection line and at least a part of the second connection line, wherein at least one end of the first connection line is configured to be connected to a first feedthrough (5, 25) and/or wherein at least one end of the second connection line is configured to be connected to a second feedthrough (5, 25).

3. Electrical arrangement according to at least one of the claims 1 or 2, wherein the antenna structure (4, 24) comprises at least a part which is straight (42) and/or a part which has a meandering shape (41).

4. Electrical arrangement according to one of the preceding claims, wherein the charging coil (3, 23), the antenna structure (4, 24) and a feedline of the antenna structure are in combination configured as an inverted F antenna or a loop antenna.

5. Electrical arrangement according to at least one of the preceding claims, wherein the antenna structure (4, 24) is configured to receive and/or transmit radiofrequency signals associated with the frequency band of the MICS spectrum.

6. Electrical arrangement according to at least one of the preceding claims, wherein the antenna structure (4, 24) is configured to receive and/or transmit radiofrequency signals associated with the frequency band of the ISM spectrum.

7. Electrical arrangement according to at least one of the preceding claims, wherein the antenna structure (4, 24) is configured to receive and/or transmit radiofrequency signals at least at a first frequency band and a second frequency band, wherein the first frequency band differs from the second frequency band.

8. Electrical arrangement according to at least one of the preceding claims 2 to 7, wherein the first connection line is connected to a receiver exchange matching circuit (214) and wherein the second connection line is connected to a receiver exchange matching circuit (214) or a transmission exchange matching circuit (215).

9. Electrical arrangement according to at least one of the preceding claims 2 to 8, wherein at least one of the first connection line or the second connection line is connected to a λ/4 impedance transmission line (213).

10. Electrical arrangement according to at least one of the preceding claims, at least one of the first connection line or the second connection line is connected to at least one of or a combination of:
- a radiofrequency transceiver (28),
- a receiver exchange matching circuit (214),
- a transmission exchange matching circuit (215), and/or
- an inductive transceiver and charging circuit (29).

11. Electrical arrangement according to claim 10, further comprising a filter network having at least one of the following components:
- passive discrete component, and/or
- a distributive transmission line.

12. Electrical arrangement according to claim 11, further comprising a plurality of capacitors (210, 211, 212, 216), wherein the capacitors are connected in series and/or in parallel to at least one of or a combination of
- the radiofrequency transceiver (28),
- the receiver exchange matching circuit (214),
- the transmission exchange matching circuit (215), and/or
- the inductive transceiver and charging circuit (29),
wherein the capacitors are configured to control at least one of or a combination of
- radiofrequency impedance of an inductive interface of the charging coil,
- resonance and/or impedance of the charging coil, and/or
- radiofrequency impedance and resonance of the antenna structure (4, 24).

13. Header structure (2, 22) for an implantable medical device (1, 21, 31) comprising electrical arrangement according to at least one of the preceding claims.

14. Header structure (2, 22) according to claim 13, wherein the header structure comprises a header body, wherein the charging coil (3, 23) and the antenna structure (4, 24) of the electrical arrangement are arranged within the header body.

15. Implantable medical device (1, 21, 31) comprising header structure (2, 22) according to claims 12 or 13, further comprising a device housing (6, 26), wherein the first feedthrough (5, 25) and/or the second feedthrough (5, 25) are arranged between header structure (2, 22) and the device housing (6, 26).

16. Implantable medical device (1, 21, 31) according to claim 15 in reference to claim 14, wherein the first connection line and/or the second connection line are electrically connected with electrical components within the device housing (6, 26) via the first feedthrough (5, 25) and/or second feedthrough (5, 25).
